# EUROPÄISCHE PATENTSCHRIFT

(11) **EP 0 716 601 B1**
(45) Veröffentlichungstag und Bekanntmachung des Hinweises auf die Patenterteilung: **28.06.2000**
(21) Anmeldenummer: 94925410.6
(22) Anmeldetag: 28.07.1994
(51) Int. Cl.: A61K 31/195, A61K 31/22

(54) **VERWENDUNG VON N,S-DIACETYLCYSTEIN-ETHYLESTER (DACEE) ZUR HERSTELLUNG EINES MEDIKAMENTS ZUR BEHANDLUNG VIRALER ERKRANKUNGEN**
USE OF N,S-DIACETYLCYSTEINE-ETHYLESTER (DACEE) FOR THE MANUFACTURE OF A MEDICAMENT FOR THE TREATMENT OF VIRAL DISEASES
UTILISATION DE N,S-DIACETYLCYSTEINE-ETHYLESTER (DACEE) POUR LA FABRICATION D'UN MEDICAMENT POUR LE TRAITEMENT DES MALADIES VIRALES

(30) Priorität: 29.07.1993 DE 4325547
(43) Veröffentlichungstag der Anmeldung: 19.06.1996
(73) Patentinhaber: Dr. Ludwig Weiss, 86438 Kissing b. München (DE)
(72) Erfinder: Dr. Ludwig Weiss, 86438 Kissing b. München (DE)
(74) Vertreter: Schüssler, Andrea, Dr.
(86) Internationale Anmeldenummer: EP9402505
(87) Internationale Veröffentlichungsnummer: WO9503792

(56) Entgegenhaltungen:
- WO-A-90/08540
- WO-A-94/14473
- FR-M- 4 619
- FR-M- 7 688
- US-A- 4 378 351
- US-A- 4 474 759
- US-A- 4 708 965
- CHEMICAL ABSTRACTS, vol. 90, no. 9, 26. Februar 1979, Columbus, Ohio, US; abstract no. 66875, 'DISINFECTANT FOR HEPATITIS B VIRUS' & JP,A,53 118 516 (AJINOMOTO CO.) 17. Oktober 1978
- LABORATORIO, Bd.40, Nr.239, 1965 Seiten 401 - 423 L.A. PUERTAS ET AL. 'ACCION DE AGENTES QUIMICOS SOBRE EL VIRUS VACUNAL'
- THE AMERICAN JOURNAL OF MEDICINE, Bd.91, Nr.3C, 1991 Seiten 122S - 130S S. DE FLORA ET AL. 'ANTIOXIDANT ACTIVITY AND OTHER MECHANISMS OF THIOLS INVOLVED IN CHEMOPREVENTION OF MUTATION AND CANCER'
- MINERVA MEDICA, Bd.69, Nr.22, 1978 Seiten 1495 - 1501 G. BOSIO ET AL. 'STUDIO CLINICO E STATISTICO SULL'IMPIEGO DI alpha-MPG NEL TRATTAMENTO DELL'EPATITE VIRALE'

## Beschreibung

Die vorliegende Erfindung betrifft eine neue Verwendung von DACEE für die Herstellung einer pharmazeutischen Zubereitung zur Behandlung Virus-induzierter Erkrankungen. Insbesondere betrifft die Erfindung eine Verwendung von DACEE für die Herstellung einer pharmazeutischen Zubereitung zur Behandlung Virus-induzierter Erkrankungen, wobei diese Verbindungen in Virus-Proteinen vorhandene Disulfid-Brücken zerstören.

Akute und chronische Infektionen von Pflanzen, Tieren und Menschen durch verschiedene virale Erreger stellen ein ernstzunehmendes medizinisches und wirtschaftliches Problem dar. Tatsächlich werden vermutlich 60% der in den industrialisierten Ländern auftretenden Krankheiten durch Viren verursacht. So zählen z.B. durch Hepatitis-Viren hervorgerufene Krankheiten weltweit zu den häufigsten Infektionen. Da die genannten Virusinfektionen im wesentlichen die Leber betreffen, kann die fortschreitende Zerstörung dieses Organs mit anschließender Entwicklung zur Zirrhose letztendlich zur Ausbildung eines hepatozellulären Carcinoms führen.

Ein wesentliches Erfordernis für ein antivirales Therapeutikum ist in einer hohen Spezifität für den viralen Erreger bei gleichzeitiger Abwesenheit bzw. Minimierung von gesundheitsschädlichen Nebenwirkungen zu sehen. Als besonders problematisch erweist sich in diesem Zusammenhang die enge Koppelung des viralen Vermehrungszyklus mit den Stoffwechsel- und Replikationsfunktionen der Wirtszelle. Gegenwärtig konzentriert sich die medizinische Forschung auf die Entwicklung von antiviralen Agenzien, die die Replikation des Virusgenoms beeinträchtigen oder verhindern, wobei insbesondere chemisch synthetisierten Nucleosid-Analoga große Bedeutung zukommt.

Eines der Hauptprobleme der antiviralen Chemotherapie ist jedoch darin zu sehen, daß für eine Vielzahl bedeutsamer Infektionserreger, wie z.B. dem Hepatitis B-Virus, keine wirksamen Substanzen zur therapeutischen Behandlung zur Verfügung stehen.

Dementsprechend liegt der vorliegenden Erfindung das technische Problem zugrunde, weitere therapeutisch wirksame und gleichzeitig pharmazeutisch verträgliche Substanzen zur Bekämpfung viraler Erkrankungen zur Verfügung zu stellen.

Die Lösung dieses technischen Problems wird dadurch erreicht, daß die in den Patentansprüchen näher gekennzeichneten Ausführungsformen bereitgestellt werden. Insbesondere wird das technische Problem dadurch gelöst, daß die vorliegende Erfindung die therapeutisch wirksame Verwendung von DACEE für die Herstellung einer pharmazeutischen Zubereitung zur Bekämpfung Virus-induzierter Erkrankungen offenbart, wobei in Virus-Proteinen vorhandene Disulfidbrücken durch diese Thiolverbindung zerstört werden.

Unter DACEE ist N,S-Diacetylcystein-ethylester zu Verstehen. Diese erfindungsgemäß verwendete Thiolverbindung ist ferner durch ihre Nicht-Toxizität im üblicherweise bei therapeutischen Behandlungen verwendeten Konzentrationsbereich (bei NAC: beispielsweise 600 mg täglich über mehrere Wochen) gekennzeichnet. DACEE wurde im medizinischen Bereich bisher als Mukolytikum eingesetzt (FR-M-4 619). Die erfindungsgemäße Thiolverbindung zeichnet sich insbesondere durch eine gute Verträglichkeit und geringe Nebenwirkungen aus. Dadurch unterscheiden sie sich besonders von bisher zur Virustherapie eingesetzten Nucleosidanaloga.

Unter dem Begriff "Virus-induzierte Erkrankung" werden Erkrankungen des tierischen und menschlichen Körpers verstanden, die durch Viren hervorgerufen oder verstärkt werden. Erfindungsgemäß sind dies insbesondere DNA-Viren, wie Hepadnaviridae, Vacciniaviridae, Parvoviridae, Papovaviridae, Adenoviridae, Poxviridae, Iridoviridae, oder RNA-Viren, wie Picornaviridae, Caliciviridae, Togaviridae, Flaviviridae, Coronaviridae, Rhabdoviridae, Filoviridae, Paramyxoviridae, Orthomyxoviridae, Bunyaviridae, Arenaviridae, Reoviridae, Birnaviridae. Im Sinne dieser Definition sind Herpesviren nicht als DNA-Viren und AIDS-Viren nicht als RNA-Viren zu verstehen. Die Erfindung betrifft bevorzugt die Verwendung von DACEE für die Bekämpfung von Virus-induzierten Erkrankungen, die durch Hepatitis-Viren, wie dem Hepatitis A, B, C oder Delta-Virus hervorgerufen werden. Insbesondere kann die Virusinduzierte Erkrankung eine chronische oder akute Hepatitis-B-Infektion oder ein hepatocelluläres Carcinom sein. Insbesondere werden unter dem Begriff "Virus-induzierte Erkrankungen" Erkrankungen verstanden, die durch Hepadnaviren, wie z.B. dem Hepatitis B Virus, dem "Woodchuck Hepatitis Virus" (WHV), dem Ground Squirrel Hepatitis Virus" (GSHV), dem "Tree Squirrel Hepatitis Virus" (TSHV) oder dem "Duck Hepatitis Virus" (DHBV) hervorgerufen werden. Ferner umfaßt die Erfindung auch die Verwendung von DACEE zur Bekämpfung pflanzlicher Erkrankungen, die durch virale Erreger, wie dem Tabakmosaikvirus (TMV) oder dem Blumenkohlmosaikvirus (CaMV) hervorgerufen werden.

Der Begriff "in Virus-Proteinen vorhandene Disulfidbrücken" bezeichnet Disulfidbrücken, die die räumliche Struktur und/oder die Funktion von viralen Proteinen durch intra- oder intermolekulare kovalente Bindungen beeinflussen. Demgemäß enthalten die Virus-Proteine, zumindest aber ein Virus-Protein der erfindungsgemäßen Verwendung von DACEE wenigstens einen Cysteinrest. Vorzugsweise enthält das Virus-Protein mehrere Cysteinreste, wie beispielsweise das HBsAg des Hepatitis B Virus. Es ist bekannt, daß andererseits Disulfidbrücken durch die Konformation des Proteins stabilisiert werden. Daher hängt die einzusetzende Menge an Thiolverbindung, die zur Spaltung der intra- oder intermolekularen Disulfidbrücken notwendig ist, von der Zugänglichkeit der jeweiligen Disulfidbrücke(n) ab.

Unter dem Begriff "Virus-Proteine" werden Proteine, vorzugsweise Hüllproteine, wie HBcAg und HBsAg, eines Virus verstanden. Insbesondere werden unter diesem Begriff Oberflächenantigen-Proteine, wie z.B. HBsAg, verstanden.

Erfindungsgemäß werden unter dem Begriff "Virus-Proteine" auch Vorläufer-Proteine für Hüllproteine verstanden, also Proteine, die nicht im endgültigen Virus vorhanden sind.

Die Erfindung betrifft ferner die Verwendung von DACEE für die Herstellung einer pharmazeutischen Zubereitung zur Behandlung Virusinduzierter Erkrankungen, wobei in Virus-Proteinen vorhandene Disulfidbrücken durch DACEE zerstört werden und die Thiolverbindung die Replikation des Virusgenoms nicht beeinflußt.

Kurze Beschreibung der Zeichnung:

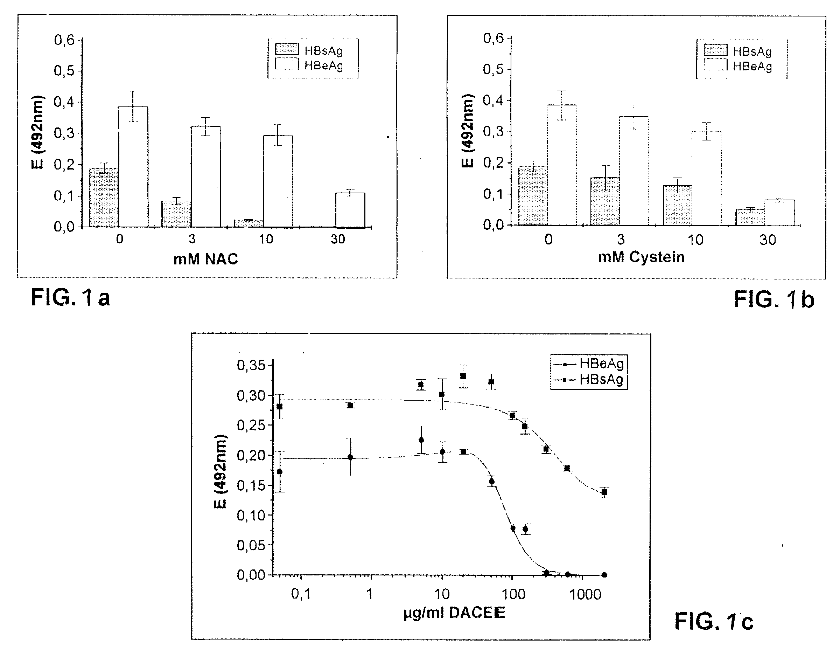

- Figur 1: zeigt den Gehalt von HBsAg und HBeAg in einem käuflichen HBsAg-positiven Serum, wobei das Serum mit unterschiedlichen Konzentrationen von NAC, Cystein bzw. DACEE inkubiert wurde, und

Die vorliegende Erfindung wird anhand der nachstehenden Beispiele näher erläutert.

### Beispiel 1: Einfluß von Thiolverbindungen auf die Replikation des Hepatitis B-Virus

Um den Einfluß von Thiolverbindungen auf die Vermehrung des Hepatitis B-Virus zu untersuchen, wurden die stabil transfizierten, potentiell infektiöse HBV-Partikel produzierenden Zellinien HepG2 2.2.15 (Sells et al., Froc. Natl. Acad. Sci. 84(1987), 1005-1009) und HepG2.4A5 (mit dem Plasmid pSPT1, 2xHBVneo (vgl. Beispiel 3) stabil transfizierte HepG2 Zellinie) verwendet. Als Thiolverbindungen wurden NAC, Cystein, Cysteinhydrochlorid und DACEE verwendet. Als Negativkontrolle wurde Cystin verwendet.

In Tabelle I ist der HBsAg- und HBeAg-Gehalt des Zellkulturüberstandes der HepG2-4A5 Zellinie, 48 Stunden nach Behandlung der Zellen mit den vorstehenden Verbindungen dargestellt. Dabei wurde der HBsAg bzw. HBeAg-Gehalt der unbehandelten Zellen willkürlich gleich 1 gesetzt und der inhibitorische Index der genannten Verbindungen in Relation dazu bestimmt. Die Antigen-Bestimmungen wurden, ebenso wie in Beispiel 2 mittels eines kommerziell erhältlichen HBsAg- bzw. HBeAg-Enzymimmuntests (Abbott-Laboratories) durchgeführt.

**Tabelle I**

| Substanz | Konz. | HBsAg | HBeAg |
|---|---|---|---|
| unbehandelt | | 0,189 ± 0,016 | 0,386 ± 0,048 |
| NAC (Vergleich) | 3 mM | 0,085 ± 0,011 | 0,323 ± 0,029 |
| | 10 mM | 0,023 ± 0,002 | 0,295 ± 0,034 |
| | 30 mM | 0,003 ± 0,001 | 0,113 ± 0,012 |
| Cystein (Vergleich) | 3 mM | 0,154 ± 0,039 | 0,348 ± 0,039 |
| | 10 mM | 0,129 ± 0,024 | 0,302 ± 0,029 |
| | 30 mM | 0,053 ± 0,006 | 0,084 ± 0,006 |

| µg/ml DACEE | | | |
|---|---|---|---|
| | 0 | 0,241 ± 0,056 | 0,310 ± 0,009 |
| | 0,05 | 0,173 ± 0,034 | 0,281 ± 0,020 |
| | 0,5 | 0,197 ± 0,031 | 0,283 ± 0,004 |
| | 5 | 0,226 ± 0,023 | 0,318 ± 0,009 |
| | 10 | 0,206 ± 0,018 | 0,302 ± 0,026 |
| | 20 | 0,206 ± 0,004 | 0,332 ± 0,019 |
| | 50 | 0,157 ± 0,009 | 0,323 ± 0,013 |
| | 100 | 0,079 ± 0,006 | 0,267 ± 0,007 |
| | 150 | 0,077 ± 0,009 | 0,249 ± 0,013 |
| | 300 | 0,004 ± 0,002 | 0,211 ± 0,007 |
| | 600 | 0,002 ± 0,001 | 0,179 ± 0,004 |
| | 2000 | 0,002 ± 0,001 | 0,139 ± 0,009 |

Die in Tabelle I dargestellten Ergebnisse wurden für NAC außerdem auch mit der Zellinie HepG2 2.2.15 und, nach transienter Transfektion von HepG2 Zellen (bzw. HuH7 Zellen), mit dem replikationskompetenten HBV-Plasmid pSPT1,2xHBV erhalten. pSPT1,2xHBV wurde wie in Beispiel 3 beschrieben hergestellt.

Sowohl für NAC- als auch Cystein-, und DACEE-behandelte Zellen konnte eine deutliche Verringerung von HBsAg im Zellkulturüberstand nachgewiesen werden. Eine graphische Darstellung vorstehender Daten findet sich in Fig. 1. Demgegenüber zeigte die Kontrollverbindung Cystin keinen Einfluß auf den Gehalt der sezernierbaren viralen Antigene, HBsAg und HBeAg (Daten nicht gezeigt).

### Beispiel 2: Untersuchungen zur Wirkungsweise von NAC, Cystein und DACEE

Um zu untersuchen, ob der antivirale Wirkungsmechanismus von NAC, Cystein bzw. DACEE auf der Zerstörung der im Virus-Protein vorhandenen Disulfidbrücken beruht, wurde der Zellkulturüberstand der HepG2-4A5-Zellinie über Nacht mit den in Tabelle II angegebenen Konzentrationen an NAC inkubiert und anschließend wurde der HBsAg-Gehalt mit einem käuflichen Testvefahren (Abbott-Laborstories) bestimmt.

Ebenso wurde käufliches HBsAg-positives Serum (Abbott-Laboratories) für 5 Min. mit den in Tabelle III angegebenen Konzentrationen von NAC, Cystein DACEE und Cysteinhydrochlorid präinkubiert und anschließend wurde der Gehalt an HBsAg und HBeAg mit vorstehendem Testverfahren bestimmt.

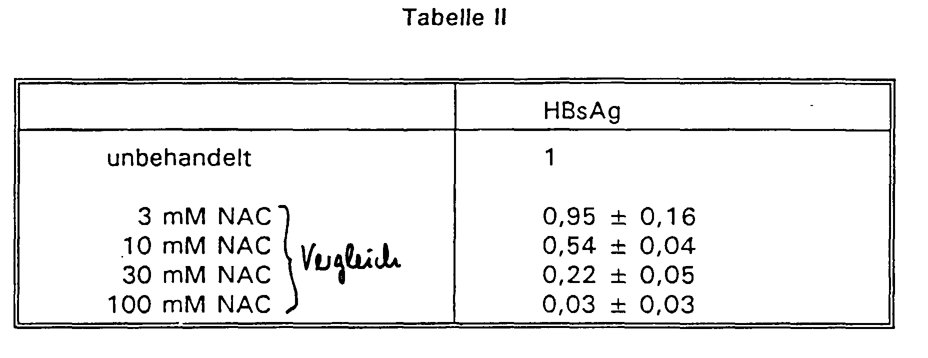

**Tabelle III**

| Substanz | Konzentration | HBsAg | HBeAg |
|---|---|---|---|
| unbehandelt | | 1,00 ± 0,05 | 1,00 ± 0,07 |
| NAC (Vergleich) | 3 mM | 0,79 ± 0,05 | 1,09 ± 0,04 |
| | 10mM | 0,49 ± 0,03 | 1,17 ± 0,04 |
| | 30 mM | 0,02 ± 0,00 | 1,27 ± 0,09 |
| Cystein (Vergleich) | 3 mM | 0,91 ± 0,02 | 1,10 ± 0,06 |
| | 10 mM | 0,68 ± 0,04 | 1,22 ± 0,05 |
| | 30 mM | 0,27 ± 0,05 | 1,21 ± 0,06 |
| DACEE | 0,6 mg/ml | 0,35 ± 0,06 | 1,11 ± 0,04 |
| | 2 mg/ml | 0,06 ± 0,00 | 1,19 ± 0,04 |
| | 6 mg/ml | 0,00 ± 0,00 | 1,09 ± 0,03 |
| Cysteinhydrochlorid (Vergleich) | 3 mM | 0,84 ± 0,004 | 1,17 ± 0,04 |
| | 10 mM | 0,33 ± 0,01 | 1,32 ± 0,05 |
| | 30 mM | 0,11 ± 0,01 | 1,30 ± 0,08 |

Tabellen II und III zeigen einen deutlichen Rückgang von HBsAg in Abhängigkeit der verwendeten Konzentrationen von NAC, DACEE bzw. Cysteinhydrochlorid. Aus der Gesamtheit dieser Daten läßt sich schließen, daß die antivirale Wirkung von NAC, Cystein, DACEE bzw. Cysteinhydrochlorid auf einer Reduktion der Disulfidbrücken im HBsAg-Komplex des Hepatitis B-Virus beruht und es damit zu einer Störung des Zusammenbaus der Hepatitis B-Virushülle kommt.

### Beispiel 3: Herstellung der Plasmide pSPT1,2xHBV und pSPT1,2xHBVneo

Zur Herstellung von pSPT1,2xHBV wurde in den mit BamHI linearisierten Klonierungsvektor pSPT19 (Boehringer Mannheim, Best.Nr.: 909815) ein aus dem Plasmid pBRHBadr4 (Fujiama et al., NAR 11 (1983), 4601-4610) stammender HBV-Anteil kloniert. Demgemäß enthält das Plasmid pSPT1,2xHBV ein HBV-Genom voller Länge (BamHI-Fragment) und zusätzlich eine terminale Redundanz von 621 bp (BamHI/Stul-Fragment). Das Plasmid pSPT1,2xHBV enthält damit den minimalen Anteil, der zur Generierung aller viralen Transkripte, inklusive der 3,5 kb langen pregonomischen RNA unbedingt notwendig ist. Dieser HBV-Anteil befindet sich ausschließlich unter der Kontrolle autologer Promotoren.

Zur Herstellung von pSPT1,2xHBVneo wurde zusätzlich in den Vektor pSPT1,2xHBV ein unter der Kontrolle des Herpes simplex TK-Promotors stehendes Neomycinresistenzgen (Neomycinanteil aus pNEO, Pharmacia Best.-Nr.: 27-4924-01) kloniert. Dabei wurde die Klonierungsstrategie so gewählt, daß der HBV-Anteil und der Neomycinresistenzanteil 3'-3' zueinander liegen, um den Einfluß des TK-Promotors auf den HBV-Anteil möglichst gering zu halten.

## Patentansprüche

1. Verwendung von N,S-Diacetylcystein-ethylester (DACEE) für die Herstellung einer pharmazeutischen Zubereitung zur Behandlung Virusinduzierter Erkrankungen.

2. Verwendung nach Anspruch 1, wobei das Virus ein DNA-oder RNA-Virus ist.

3. Verwendung nach Anspruch 1 oder 2, wobei das Virus ein Hepatitis-Virus ist.

4. Verwendung nach Anspruch 1 oder 2, wobei das Virus ein Hepadna-Virus ist.

5. Verwendung nach Anspruch 3 oder 4, wobei das Virus das Hepatitis-B-Virus ist.

6. Verwendung nach Anspruch 3, wobei das Virus das Hepatitis-Delta-Virus ist.

7. Verwendung nach einem der Ansprüche 1 bis 6, wobei die Virus-induzierte Erkrankung eine chronische oder akute Hepatitis-B-Infektion oder ein hepatozelluläres Carcinom ist.

## Claims

1. Use of N,S-diacetylcysteine ethyl ester (DACEE) for the manufacture of a pharmaceutical preparation for the treatment of viral diseases.

2. Use according to claim 1, wherein the virus is a DNA or RNA virus.

3. Use according to claim 1 or 2, wherein the virus is a hepatitis virus.

4. Use according to claim 1 or 2, wherein the virus is a hepadnavirus.

5. Use according to claim 3 or 4, wherein the virus is the hepatitis B virus.

6. Use according to claim 3, wherein the virus is the hepatitis delta virus.

7. Use according to any one of claims 1 to 6, wherein the viral disease is a chronic or acute hepatitis B infection or a hepatocellular carcinoma.

## Revendications

1. Utilisation de l'ester éthylique de N,S-diacétylcystéine (DACEE) pour la fabrication d'une préparation pharmaceutique pour le traitement de maladies induites par un virus.

2. Utilisation selon la revendication 1, dans laquelle le virus est un virus à ADN ou à ARN.

3. Utilisation selon la revendication 1 ou 2, dans laquelle le virus est un virus de l'hépatite.

4. Utilisation selon la revendication 1 ou 2, dans laquelle le virus est un virus de la famille des Hépadnaviridés.

5. Utilisation selon la revendication 3 ou 4, dans laquelle le virus est le virus de l'hépatite B.

6. Utilisation selon la revendication 3, dans laquelle le virus est le virus de l'hépatite delta.

7. Utilisation selon l'une des revendications 1 à 6, dans laquelle la maladie induite par un virus est une infection chronique ou aigué par l'hépatite B ou un carcinome hépatocellulaire.
